# EUROPEAN PATENT SPECIFICATION

(11) **EP 4 038 180 B1**
(45) Date of publication and mention of the grant of the patent: **03.01.2024**
(21) Application number: 20797035.1
(22) Date of filing: 02.10.2020
(51) Int. Cl.: A61K 48/00, C07K 14/80, C12N 9/02, C12N 15/86, C12N 5/0789, A61K 35/28, A61K 38/00

(54) **TREATMENT OF CHRONIC GRANULOMATOUS DISEASE**
BEHANDLUNG VON CHRONISCHER GRANULOMATÖSER ERKRANKUNG
TRAITEMENT DE LA GRANULOMATOSE SEPTIQUE CHRONIQUE

(30) Priority: 02.10.2019 EP 19201180
(43) Date of publication of application: 10.08.2022
(73) Proprietor: Universität Zürich, 8006 Zürich (CH)
(72) Inventor: SILER, Ulrich, Wolfgang, 68220 Leymen (FR); REICHENBACH, Janine, 8032 Zürich (CH); HÄNSELER, Walther, 8400 Winterthur (CH)
(74) Representative: Vossius & Partner Patentanwälte Rechtsanwälte mbB
(86) International application number: PCT/EP2020/077638
(87) International publication number: WO 2021/064164

(56) References cited:
- WO-A1-2018/045266
- CHRISTIAN BRENDEL ET AL: "Human miR223 Promoter as a Novel Myelo-Specific Promoter for Chronic Granulomatous Disease Gene Therapy", HUMAN GENE THERAPY METHODS, vol. 24, no. 3, 1 June 2013 (2013-06-01), pages 151-159, XP055677547, ISSN: 1946-6536, DOI: 10.1089/hgtb.2012.157
- MARIA CHIRIACO ET AL: "Dual-regulated Lentiviral Vector for Gene Therapy of X-linked Chronic Granulomatosis", MOLECULAR THERAPY : THE JOURNAL OF THE AMERICAN SOCIETY OF GENE THERAPY, vol. 22, no. 8, 1 August 2014 (2014-08-01) , pages 1472-1483, XP055492876, US ISSN: 1525-0016, DOI: 10.1038/mt.2014.87
- DREYER ANNE-KATHRIN ET AL: "TALEN-mediated functional correction of X-linked chronic granulomatous disease in patient-derived induced pluripotent stem cells", BIOMATERIALS, ELSEVIER, AMSTERDAM, NL, vol. 69, 3 August 2015 (2015-08-03), pages 191-200, XP029262036, ISSN: 0142-9612, DOI: 10.1016/J.BIOMATERIALS.2015.07.057

## Description

### Background of the invention

Chronic granulomatous disease (CGD) comprises a group of inborn immunodeficiencies with an incidence between 1/19.230 in Saudi Arabia (Suliaman et al. (2009) Pediatric Asthma, Allergy & Immunology 22, 21-26) and 1/450.000 in Sweden (Ahlin et al. (1995) Acta Paediatr 84, 1386-1394). The disease manifests early in life as the body of affected patients is not able to protect itself from bacterial and fungal infections. Life expectancy is strongly compromised despite life-long prophylactic treatment with antibiotics and antimycotics (Kuhns et al. (2010) New Engl J Med 363 (27) 2600-10; van den Berg et al. (2009) PloS one 4(4): e5234). The primary cause of the disease is a strongly diminished or absent ability of phagocytes (granulocytes, monocytes and macrophages) to produce reactive oxygen species (ROS) for pathogen inactivation, due to a defect within the phagocytic NADPH (nicotinamide adenine dinucleotide phosphate) oxidase enzyme complex. The NADPH oxidase enzyme complex consists of six subunits (gp91phox, p22phox, p40phox, p47phox, p67phox and Rac2). In about 60% of all CGD patients, the disease is caused by mutations within the gp91phox subunit encoding gene (*CYBB*) on the X chromosome and therefore termed X-CGD. Another -30% of patients present with autosomal-recessive *NCF1* gene (p47phox subunit) alterations (p47phox-deficient form of CGD), whilst only 5% of the cases are due to autosomal-recessive mutations in p67phox and p22phox (Roos et al. (2003) Microbes Infect 5: 1307-15).

Gene therapy is a new treatment option for patients suffering from monogenic diseases. The principal of present state-of-the-art gene therapy is to introduce a functional version of the mutated gene into the genome of affected cells. Current clinical approaches mainly rely on delivery of the therapeutic gene by adeno-associated virus-based vectors or by retroviral (gamma-retroviral or lentiviral) vectors. The inserted functional version of the mutated gene (transgene) results in the expression of the new gene product, which compensates for the absence of the natural gene product. Since first clinical success in the year 2000, the number of diseases for which clinical success has been achievable by this gene addition approach of gene therapy has been continuously rising.

Latest developments of designer nucleases (Zinc-finger nucleases (ZFNs), Transcription activator-like effector nucleases (TALENs), Clustered Regularly Interspaced Short Palindromic Repeats CRISPR/Cas methodology) allow for sequence-specific gene modification (deletion or repair) as well as for sequence-specific insertion of DNA. Sequence-specific gene disruption for therapeutic purposes has already found clinical application (TALENs: W.Quasim et al. (2015) Blood 126 : 2016; ZNFs: ClinicalTrials.gov Identifier NCT02500849 and CRISPR/Cas9: Cyranoski D. et al. Nature 539, 479; ClinicalTrials.gov Identifier: NCT03057912). Currently, only zinc-finger nucleuses are clinically evaluated for sequence specific insertion in order to cure hemophilia B, MPS I and MPS II (https://www.sangamo.com/product-pipeline).

At present the only curative therapy for CGD is allogeneic hematopoietic stem cell (HSC) transplantation from a human leukocyte antigen (HLA)-identical donor. For up to about 75% of patients of non-consanguineous European descent an HLA-identical donor can be identified (Grager et al. (2014) N Engl J Med 371: 339-48). For the remaining 25%, gene therapy-based correction of autologous HSCs represents an additional potentially curative treatment option.

Gene therapy for CGD is therefore a reasonable option for patients lacking HLA-identical HSC donors. As learned from female carriers of X-linked CGD, an NADPH oxidase activity above 20% can be associated with a healthy phenotype (Bjorgvinsdottir et al. (1997) Blood 89, 41-48; Kuhns et al. (2010) N Engl J Med 363, 2600-2610). Therefore, gene therapeutic correction levels of ca. 20% NAPDH oxidase activity are considered sufficient to achieve clinical success.

The first clinically successful phase I/II gene therapy trial for X-CGD patients was initiated by University Children's Hospital Zurich and Johann Wolfgang Goethe University Hospital Frankfurt in 2004 (NCT 00927134; NCT 00564759). Within this trial two children were treated in Zurich and two adult patients were treated in Frankfurt. The gene therapy vector consisted of an LTR-driven (non-self-inactivating (SIN)) gamma-retroviral vector with a gp91phox-encoding cDNA as transgene, resulting in ubiquitous transgene expression. The study was clinically successful, as all four patients recovered from pre-existing treatment refractory infections (Ott et al. (2006) Nat Med 12: 401-409; Siler et al. (2015) Curr Gene Ther 15: 416-427; Bianchi et al. (2009) Blood 114: 2619-2622). Long-term follow up however, revealed two serious adverse events (SAEs) leading to closure of the trial in 2010:
- Transactivation: In clones in which the viral vector had integrated adjacent to the *MDS1-EVI1* gene, enhancer sequences present in the long-terminal repeat of the gamma-retroviral vector interacted with the promoter of the *MDS1-EVI1* proto-oncogene (Evi1 transactivation), which led to a selective advantage of these HSC clones. This caused clonal dominance, and genomic instability with monosomy 7 and development of myelodysplastic syndrome (MDS) in three out of four gene therapy treated patients.
- Silencing: The promoter of the viral vector became progressively methylated, which led to decreasing expression of the therapeutic transgene (transgene silencing), and to the re-appearance of CGD.

These SEAs mandated the development of safer gene therapy vectors, based on lentiviral self-inactivated (SIN) vectors with
(I) a different integration profile, e.g. the use of different viral vector, to prevent integration of the viral vector in proximity to the *MDS1-EVI1* gene,
(II) tissue-specific transgene expression, i.e. only in myeloid-derived cells that need transgene expression, but not in HSC, to prevent potential selective advantage of HSC clones in case of transactivation, and
(III) prevention of silencing of transgene expression over time to facilitate long term efficacy.

Brendel et al. discloses miR223 promoter-driven p47phox gene expression from a gammaretroviral vector in CD34+ haematopoietic stem cells. Lentiviral vectors expressing the gp47phox gene under the control of the miR223-promoter in monocytes are also disclosed.

Chiriaco et al. discloses myeloid-specific promoter (minimal promoter of gp91 phox fused to the SP146 synthetic enhancer/promoter)-driven gp91phox gene expression from a lentiviral vector in CD34+ haematopoietic stem cells. Lentiviral vectors expressing the gp91phox gene under the control of the miR223-promoter are also disclosed.

### Terms and definitions

The term "lentiviral vector" when used in the context of the present specification refers to a virion or virus genome derived from a lentivirus and adapted for human gene therapy. It comprises a transgene including a coding sequence and a promoter operable in a human patient's cell. Lentiviral gene therapy is well known to the skilled artisan; recent scientific publications on the topic include Milone and O'Doherty, (2018) Leukemia 32, 1529-1541; Yáñez-Muñoz et al. (2006) Nature Medicine 12, 348-353; Aiuti et al. (2013), Science 341(6148), DOI: 10.1126/science.1233151).

The term "functional variant" when used in the context of the present specification refers to a variant of a gene encoding a polypeptide, wherein a dysfunctional or non-functional variant of this gene is present in the patient, but the dysfunctional or non-functional variant does not serve a particular biological function within the patient. In stimulated phagocytes of healthy individuals, gp91phox protein (encoded by *CYBB* gene), p22phox protein (encoded by *CYBA* gene), p47phox protein (encoded by *NCF1* gene), p40phox protein (encoded by *NCF4* gene), p67phox protein (encoded by *NCF2* gene) together form one enzyme complex termed NADPH oxidase. This NADPH oxidase has catalytic activity and is mandatory for phagocytic production of reactive oxygen species (ROS). The Rac2 protein (encoded by RAC2 gene) is required to induce the formation of the NADPH oxidase enzyme complex upon cell stimulation. Mutations in any of these genes can result in the synthesis in misfolded proteins or in the absence of one gene product, which finally results in the inability of patient cells to form an active NADPH oxidase complex and hence in their inability to produce ROS, causing the CGD phenotype. The functional variant as encoded by a gene therapy vector is capable of complementing or replacing the dysfunctional or non-functional variant and restore NADPH oxidase function.

### Summary of the invention

Based on this background, it is the objective of the present invention to provide efficient means for the treatment of a genetic disease in chronic granulomatous disease.

This objective is attained by the subject-matter of the independent claims, with further advantageous features set forth in the dependent claims.

A first aspect of the invention relates to an isolated human hematopoietic stem cell (HSC) or progenitor cell, particularly a CD-34 positive hematopoietic stem cell, wherein the stem cell or the progenitor cell is able to differentiate into a myeloid cell, transduced with a lentiviral vector.

The lentiviral vector comprises
- a nucleic acid sequence encoding a functional variant of a dysfunctional polypeptide selected from the group comprising gp91 phox, p22phox, p40phox, p47phox, p67phox and Rac2; under transcriptional control of, and
- a promoter sequence operable in a human cell, wherein the promoter comprises or essentially consists of the miR223 promoter sequence (SEQ ID NO 01).
miR223 promoter sequence (SEQ ID NO 01)

ACTTGTACAGCTTCACAGGGCTCCATGCTTAGAAGGACCCCACACTTAGTTTAATGTTCT
GCTGTCATCATCTTGATATTCTTAATTTTTAAATAAAGGGCCTATCGTTTTCATTTTTTACT
GGGCCTTGCAAATTATGTAGCTGGTTCTGTATGCCAGGAGAGAAGTTGGAAGTAAAATG
GTATTCCAGGACCAGGAGGCATTCTGGCAGAGTGAAAGAACATGTGATTTGGAGTCCAT
GGGGATGGGTTTAAATTTCAGCTTTCCACTAATTTGCTTTGTGATACTGAGTATTTCCTTT
TATCCCTCAGAGGCTCTGTTTCTCAATTTTGACTACGGGTTTTTTCATTAGATAATGTCTC
AGTTCTGGTATTCCAGGTTTCCCTCAATTATTCTGGGAAAACCTCCTTGACCCACAGGCA
GAGCCTAGGGCAGCCAGGTGCTTTCTACTCTCTCTCTCTCTGCAGCTTGGAAAGTTAGT
GTCTGTTGAAGGTCAGCTGGGAGTTGGTGGAGGCAGGGCAGTGGCCTGCTACTATTGC
TGCAGTAGCAGACCCTTTCACAACAGCATTGTTTTGTCATTTTGCATCCAGATTTCCGTT
GGCTAACCTCAGTCTTATCTTCCTCATTTCTGTTTCCTGTTGAAGACACCAAGGGCCCTT
CAAAACACAGAAGCTTCTTGCTCACGGCAGAAAGCCCAATTCCATCTGGCCCCTGCAG
GTTGGCTCAGCACTGGGGAATCAGAGTCCCCTCCATGACCAAGGCACCACTCCACTGA
CAG.

In certain embodiments, the cell is provided for treatment of chronic granulomatous disease, and said vector comprises a nucleic acid sequence encoding a functional variant or functional variants of gp91phox (UniProt number P04839), p22phox (UniProt number P13498), p40phox UniProt number Q15080)), p47phox UniProt number P14598), p67phox UniProt number P19878) and/or Rac2 (UniProt number P15153).

In certain embodiments, the vector does not comprise lentiviral promoter/enhancer elements. The term "enhancer element" in the context of the present specification refers to a short nucleic acid sequence that can be bound by activator proteins to increase the likelihood that transcription of a particular sequence, particularly a promoter sequence, will occur.

The gene therapy vector of the inventior, is based on or derived from a lentiviral vector.

In one embodiment of the gene therapy vector of the invention, the vector comprises or essentially consists of a nucleic acid characterized by SEQ ID 02.
SEQ ID No 02:
Lenti_mir223_p47phox_PRE4_KAN(UZH1p47CGD) 8688bp (sequence elements in 5' to 3' direction indicated by font:)

Lowercase font: CMV promoter; lowercase underscored: Lentiviral LTR: 5'U5; lowercase italics: Lentiviral Leader Sequence; uppercase font: miR223 Promoter; uppercase underscore: Kozak Sequence; uppercase italics: p47phox Open Reading Frame; lowercase italics underscored: restriction sites; lowercase: WPRE element; lowercase italics underscored: restriction sites; lowercase underscored: Lentiviral LTR: 3'U3; lowercase italics: Lentiviral LTR: 3'R; lowercase italics bold: Lentiviral LTR: 3'U5; lowercase:

ccattgcatacgttgtatccatatcataatatgtacatttatattggctcatgtccaacattaccgccatgttgacattgattattgactag
ttattaatagtaatcaattacggggtcattagttcatagcccatatatggagttccgcgttacataacttacggtaaatggcccgcctg
gctgaccgcccaacgacccccgcccattgacgtcaataatgacgtatgttcccatagtaacgccaatagggactttccattgacg
tcaatgggtggagtatttacggtaaactgcccacttggcagtacatcaagtgtatcatatgccaagtacgccccctattgacgtcaa
tgacggtaaatggcccgcctggcattatgcccagtacatgaccttatgggactttcctacttggcagtacatctacgtattagtcatc
gctattaccatggtgatgcggttttggcagtacatcaatgggcgtggatagcggtttgactcacggggatttccaagtctccacccc
attgacgtcaatgggagtttgttttggcaccaaaatcaacgggactttccaaaatgtcgtaacaactccgccccattgacgcaaat
gggcggtaggcgtgtacggtgggaggtctatataagcagagctcgtttagtgaaccggggtctctctggttagaccagatctgag
cctgggagctctctggctaactagggaacccactgcttaagcctcaataaagcttgccttgagtgcttcaagtagtgtgtgcccgtc
tgttgtgtgactctggtaactagagatccctcagacccttttagtcagtgtggaaaatctctagcagtggcgcccgaacagggactt
gaaagcgaaagggaaaccagaggagctctctcgacgcaggactcggcttgctgaagcgcgcacggcaagaggcgaggg
gcggcgactggtgagtacgccaaaaattttgactagcggaggctagaaggagagagatgggtgcgagagcgtcagtattaag
cgggggagaattagatcgcgatgggaaaaaattcggttaaggccagggggaaagaaaaaatataaattaaaacatatagtat
gggcaagcagggagctagaacgattcgcagttaatcctggcctgttagaaacatcagaaggctgtagacaaatactgggaca
gctacaaccatcccttcagacaggatcagaagaacttagatcattatataatacagtagcaaccctctattgtgtgcatcaaagga
tagagataaaagacaccaaggaagctttagacaagatagaggaagagcaaaacaaaagtaagaccaccgcacagcaag
cggccgctgatcttcagacctggaggaggagatatgagggacaattggagaagtgaattatataaatataaagtagtaaaaatt
gaaccattaggagtagcacccaccaaggcaaagagaagagtggtgcagagagaaaaaagagcagtgggaataggagctt
tgttccttgggttcttgggagcagcaggaagcactatgggcgcagcgtcaatgacgctgacggtacaggccagacaattattgtc
tggtatagtgcagcagcagaacaatttgctgagggctattgaggcgcaacagcatctgttgcaactcacagtctggggcatcaa
gcagctccaggcaagaatcctggctgtggaaagatacctaaaggatcaacagctcctggggatttggggttgctctggaaaact
catttgcaccactgctgtgccttggaatgctagttggagtaataaatctctggaacagatttggaatcacacgacctggatggagtg
ggacagagaaattaacaattacacaagcttaatacactccttaattgaagaatcgcaaaaccagcaagaaaagaatgaacaa

gaattattggaattagataaatgggcaagtttgtggaattggtttaacataacaaattggctgtggtatataaaattattcataatgata
gtaggaggcttggtaggtttaagaatagtttttgctgtactttctatagtgaatagagttaggcagggatattcaccattatcgtttcaga
cccacctcccaaccccgaggggacccgacaggcccgaaggaatagaagaagaaggtggagagagagacagagacaga
tccattcgattagtgaacggatctcgacggtatcggttaacttttaaaagaaaaggggggattggggggtacagtgcaggggaa
agaatagtagacataatagcaacagacatacaaactaaagaattacaaaaacaaattacaaaaattcaaaattttatcgatca
cgagactagcctcgagaagcttgatataatagtcgacACTTGTACAGCTTCACAGGGCTCCATGCTTAGA
AGGACCCCACACTTAGTTTAATGTTCTGCTGTCATCATCTTGATATTCTTAATTTTTAAAT
AAAGGGCCTATCGTTTTCATTTTTTACTGGGCCTTGCAAATTATGTAGCTGGTTCTGTAT
GCCAGGAGAGAAGTTGGAAGTAAAATGGTATTCCAGGACCAGGAGGCATTCTGGCAGA
GTGAAAGAACATGTGATTTGGAGTCCATGGGGATGGGTTTAAATTTCAGCTTTCCACTA
ATTTGCTTTGTGATACTGAGTATTTCCTTTTATCCCTCAGAGGCTCTGTTTCTCAATTTTG
ACTACGGGTTTTTTCATTAGATAATGTCTCAGTTCTGGTATTCCAGGTTTCCCTCAATTA
TTCTGGGAAAACCTCCTTGACCCACAGGCAGAGCCTAGGGCAGCCAGGTGCTTTCTAC
TCTCTCTCTCTCTGCAGCTTGGAAAGTTAGTGTCTGTTGAAGGTCAGCTGGGAGTTGGT
GGAGGCAGGGCAGTGGCCTGCTACTATTGCTGCAGTAGCAGACCCTTTCACAACAGCA
TTGTTTTGTCATTTTGCATCCAGATTTCCGTTGGCTAACCTCAGTCTTATCTTCCTCATTT
CTGTTTCCTGTTGAAGACACCAAGGGCCCTTCAAAACACAGAAGCTTCTTGCTCACGGC
AGAAAGCCCAATTCCATCTGGCCCCTGCAGGTTGGCTCAGCACTGGGGAATCAGAGTC
CCCTCCATGACCAAGGCACCACTCCACTGACAGGGATCCAAGCTTGCCACCATGGGC
GACACCTTCATCCGGCACATCGCCCTGCTGGGCTTCGAGAAGCGGTTCGTGCCCAGC
CAGCACTACGTGTACATGTTTCTGGTGAAGTGGCAGGACCTGAGCGAGAAGGTGGTGT
ACCGGCGGTTCACCGAGATCTACGAGTTCCACAAGACCCTGAAAGAGATGTTCCCCAT
CGAGGCCGGAGCCATCAACCCCGAGAACCGGATCATCCCCCACCTGCCTGCCCCCAA
GTGGTTCGACGGCCAGAGAGCCGCCGAGAACAGGCAGGGCACCCTGACCGAGTACT
GCAGCACCCTGATGAGCCTGCCCACCAAGATCAGCCGGTGCCCTCACCTGCTGGATTT
CTTCAAAGTGCGGCCCGACGACCTGAAGCTGCCCACCGACAACCAGACCAAGAAGCC
CGAGACCTACCTGATGCCCAAGGACGGCAAGAGCACCGCCACCGACATCACCGGCCC
CATCATCCTGCAGACCTACCGGGCCATCGCCGACTACGAGAAAACCAGCGGCAGCGA
GATGGCCCTGAGCACCGGCGACGTGGTGGAGGTGGTGGAAAAGAGCGAGAGCGGGT
GGTGGTTCTGCCAGATGAAGGCCAAGCGGGGCTGGATTCCCGCCAGCTTCCTGGAAC
CCCTGGACAGCCCCGACGAGACCGAGGACCCCGAGCCCAACTACGCCGGCGAGCCC
TACGTGGCCATCAAGGCCTACACCGCCGTGGAGGGCGACGAGGTGTCCCTGCTGGAA
GGCGAGGCCGTGGAGGTGATCCACAAGCTGCTGGACGGATGGTGGGTGATCCGGAA
GGACGACGTGACCGGCTACTTCCCCAGCATGTACCTGCAGAAGAGCGGCCAGGACGT
CAGCCAGGCCCAGCGGCAGATCAAGAGAGGCGCCCCTCCCAGGCGGAGCAGCATCC
GGAACGCCCACAGCATCCACCAGAGAAGCCGGAAGAGACTGAGCCAGGACGCCTACC
GGCGGAACAGCGTGCGGTTCCTGCAGCAGCGGCGGAGGCAGGCCAGACCCGGCCCT

CAGAGCCCCGGCAGCCCCCTGGAAGAGGAACGGCAGACCCAGCGGAGCAAGCCCCA
GCCCGCCGTGCCCCCTAGACCCAGCGCCGACCTGATCCTGAACCGGTGCAGCGAGAG
CACCAAGCGGAAGCTGGCCAGCGCCGTGTGATGActcgagtattatatcaagcttatcgataccgtcgaa
tcccccgggctgcaggaattcgagcatcttaccgccatttattcccatatttgttctgtttttcttgatttgggtatacatttaaatgttaata
aaacaaaatggtggggcaatcatttacatttttagggatatgtaattactagttcaggtgtattgccacaagacaaacatgttaaga
aactttcccgttatttacgctctgttcctgttaatcaacctctggattacaaaatttgtgaaagattgactgatattcttaactatgttgctcc
ttttacgctgtgtggatatgctgctttaatgcctctgtatcatgctattgcttcccgtacggctttcgttttctcctccttgtataaatcctggtt
gctgtctctttatgaggagttgtggcccgttgtccgtcaacgtggcgtggtgtgctctgtgtttgctgacgcaacccccactggctggg
gcattgccaccacctgtcaactcctttctgggactttcgctttccccctcccgatcgccacggcagaactcatcgccgcctgccttgc
ccgctgctggacaggggctaggttgctgggcactgataattccgtggtgttgtcggggaagggcctgctgccggctctgcggcct
cttccgcgtcttcgccttcgccctcagacgagtcggatctccctttgggccgcctccccgcctggaattcgagctcggtacctttaag
accaatgacttacaaggcagctgtagatcttagccactttttaaaagaaaaggggggactggaagggctaattcactcccaacg
aagacaagatctgctttttgcttgtactgggtctctctggttagaccagatctgagcctgggagctctctggctaactagggaaccta
ctgcttaagcctcaataaagcttgccttgagtgcttcaagtagtgtgtgcccgtctgttgtgtgactctggtaactagagatc
cctcagacccttttagtcagtgtggaaaatctctagcagtagtagttcatgtcatcttattattcagtatttataacttgcaaagaa
atgaatatcagagagtgagaggaacttgtttattgcagcttataatggttacaaataaagcaatagcatcacaaatttcacaaata
aagcatttttttcactgcattctagttgtggtttgtccaaactcatcaatgtatcttatcatgtctggctctagctatcccgcccctaactcc
gcccatcccgcccctaactccgcccagttccgcccattctccgccccatggctgactaattttttttatttatgcagaggccgaggcc
gcctcggcctctgagctattccagaagtagtgaggaggcttttttggaggcctagggacgtacccaattcgccctatagtgagtcgt
attacgcgcgctcactggccgtcgttttacaacgtcgtgactgggaaaaccctggcgttacccaacttaatcgccttgcagcacat
ccccctttcgccagctggcgtaatagcgaagaggcccgcaccgatcgcccttcccaacagttgcgcagcctgaatggcgaatg
ggacgcgccctgtagcggcgcattaagcgcggcgggtgtggtggttacgcgcagcgtgaccgctacacttgccagcgccctag
cgcccgctcctttcgctttcttcccttcctttctcgccacgttcgccggctttccccgtcaagctctaaatcgggggctccctttagggttc
cgatttagtgctttacggcacctcgaccccaaaaaacttgattagggtgatggttcacgtagtgggccatcgccctgatagacggtt
tttcgccctttgacgttggagtccacgttctttaatagtggactcttgttccaaactggaacaacactcaaccctatctcggtctattctttt
gatttataagggattttgccgatttcggcctattggttaaaaaatgagctgatttaacaaaaatttaacgcgaattttaacaaaatatta
acgcttacaatttaggtggcacttttcggggaaatgtgcgcggaacccctatttgtttatttttctaaatacattcaaatatgtatccgct
catgagacaataaccctgataaatgcttcaataatagcacctagatcaagagacaggatgaggatcgtttcgcatgattgaaca
agatggattgcacgcaggttctccggccgcttgggtggagaggctattcggctatgactgggcacaacagacaatcggctgctct
gatgccgccgtgttccggctgtcagcgcaggggcgcccggttctttttgtcaagaccgacctgtccggtgccctgaatgaactgca
agacgaggcagcgcggctatcgtggctggccacgacgggcgttccttgcgcagctgtgctcgacgttgtcactgaagcgggaa
gggactggctgctattgggcgaagtgccggggcaggatctcctgtcatctcaccttgctcctgccgagaaagtatccatcatggct
gatgcaatgcggcggctgcatacgcttgatccggctacctgcccattcgaccaccaagcgaaacatcgcatcgagcgagcac
gtactcggatggaagccggtcttgtcgatcaggatgatctggacgaagagcatcaggggctcgcgccagccgaactgttcgcc
aggctcaaggcgagcatgcccgacggcgaggatctcgtcgtgacccatggcgatgcctgcttgccgaatatcatggtggaaaa
tggccgcttttctggattcatcgactgtggccggctgggtgtggcggaccgctatcaggacatagcgttggctacccgtgatattgct
gaagagcttggcggcgaatgggctgaccgcttcctcgtgctttacggtatcgccgctcccgattcgcagcgcatcgccttctatcg

ccttcttgacgagttcttctgaattattaacgcttacaatttcctgatgcggtattttctccttacgcatctgtgcggtatttcacaccgcatc
aggtggcacttttcggggaaatgtgcgcggaacccctatttgtttatttttctaaatacattcaaatatgtatccgctcatgaccaaaat
cccttaacgtgagttttcgttccactgagcgtcagaccccgtagaaaagatcaaaggatcttcttgagatcctttttttctgcgcgtaat
ctgctgcttgcaaacaaaaaaaccaccgctaccagcggtggtttgtttgccggatcaagagctaccaactctttttccgaaggtaa
ctggcttcagcagagcgcagataccaaatactgttcttctagtgtagccgtagttaggccaccacttcaagaactctgtagcaccg
cctacatacctcgctctgctaatcctgttaccagtggctgctgccagtggcgataagtcgtgtcttaccgggttggactcaagacgat
agttaccggataaggcgcagcggtcgggctgaacggggggttcgtgcacacagcccagcttggagcgaacgacctacaccg
aactgagatacctacagcgtgagctatgagaaagcgccacgcttcccgaagggagaaaggcggacaggtatccggtaagc
ggcagggtcggaacaggagagcgcacgagggagcttccagggggaaacgcctggtatctttatagtcctgtcgggtttcgcca
cctctgacttgagcgtcgatttttgtgatgctcgtcaggggggcggagcctatggaaaaacgccagcaacgcggcctttttacggtt
cctggccttttgctggccttttgctcacatgttctttcctgcgttatcccctgattctgtggataaccgtattaccgcctttgagtgagctgat
accgctcgccgcagccgaacgaccgagcgcagcgagtcagtgagcgaggaagcggaagagcgcccaatacgcaaaccg
cctctccccgcgcgttggccgattcattaatgcagctggcacgacaggtttcccgactggaaagcgggcagtgagcgcaacgc
aattaatgtgagttagctcactcattaggcaccccaggctttacactttatgcttccggctcgtatgttgtgtggaattgtgagcggata
acaatttcacacaggaaacagctatgaccatgattacgccaagcgcgcaattaaccctcactaaagggaacaaaagctggag
ctgcaagcttgg

The stem or progenitor cell is obtained from a patient suffering from CGD and transduced with the gene therapy vector of the invention.

In certain embodiments, the isolated human myeloid stem cell or progenitor cell according to the invention is a human CD-34 positive cell. Whilst long-term repopulating hematopoietic stem cells are enriched in human CD34+ bone marrow cell population, cells with long-term engraftment capabilities of normal adult mice are Lin-, c-kit+, Sca-1+ and CD34- (Sato T., Laver J.H. & Ogawa M. (1999) Blood 94: 2548-2554). Human and mouse stem cells differ not just in surface marker expression, but also in growth conditions (medium, cytokines...) for cell expansion and for viral transduction.

A further aspect of the disclosure relates to a method of manufacturing a human progenitor cell able to differentiate into a monocyte or macrophage cell. The method comprises the steps of:
- providing ex-vivo a CD34+ HSC obtained from a patient suffering from CGD, and
- transducing said CD34+ HSC with a lentiviral vector comprising
   ∘ a coding nucleic acid sequence encoding a functional variant of a polypeptide selected from gp91phox, p22phox, p40phox, p47phox, p67phox and Rac2; the coding sequence being under transcriptional control of
   ∘ a promoter sequence operable in a human cell, particularly in a myeloid cell, wherein said promoter sequence comprises or essentially consists of the miR223 promoter sequence (SEQ ID NO 01).

The invention also relates to a method of treatment of a patient diagnosed with chronic granulomatous disease. This method comprises the steps of
- providing a CD34+ HSC isolated from the patient,
- transducing this CD34+ HSC with a lentiviral vector comprising
   ∘ a coding nucleic acid sequence encoding a functional variant of a polypeptide selected from gp91 phox, p22phox, p40phox, p47phox, p67phox and Rac2; the coding sequence being under transcriptional control of
   ∘ a promoter sequence operable in a human cell, particularly in a myeloid cell, wherein said promoter sequence comprises or essentially consists of the miR223 promoter sequence (SEQ ID NO 01)
- administering said CD34+ HSC to the patient.

The patient's native nucleic acid sequence encoding the dysfunctional polypeptide, which causes CGD, is complemented by a "healthy" nucleic acid sequence, encoding a functional polypeptide by transducing the CD34+ cell with the vector of the invention. The so transduced (and complemented) HSC or progenitor cell is then transferred back into the patient.

The vector of the invention may be used to transduce autologous CD34+ HSC or hematopoietic progenitor cells able to differentiate into myeloid cells such as e.g. monocytes, macrophages, or neutrophils. The CD34+ cells are obtained from the patient suffering from the genetic disease. The transduced HSC are transferred back to the patient in order to undergo division and differentiation into myeloid cells.

The genetic disease treated by the invention is chronic granulomatous disease, and the vector or the HSC or hematopoietic progenitor cell able to differentiate into a myeloid cell, in particular a monocyte/macrophage or neutrophil, comprises a nucleic acid sequence encoding a functional variant or functional variants of a gene selected from the group comprising gp91phox, p22phox, p40phox, p47phox, p67phox and Rac2. The HCS or hematopoietic progenitor cell comprises or is transduced by the vector.

According to a further aspect of the invention, a method for correcting the CGD phenotype in myeloid cells is provided. The method comprises providing a HCS or hematopoietic progenitor cell of the invention and administering the HCS or hematopoietic progenitor cell of the invention to a patient suffering from the disease.

In certain embodiments, the HCS or hematopoietic progenitor cell of the invention is provided by obtaining a HCS or hematopoietic progenitor cell from the patient suffering from CGD and transducing the HCS or hematopoietic progenitor cell with the vector as specified herein.

The genetic disease in myeloid cells is CGD, and the HCS or hematopoietic progenitor cell obtained from the patient comprises or is transduced with a vector comprising a nucleic acid sequence encoding a functional variant or functional variants of gp91 phox, p22phox, p40phox, p47phox, p67phox and/or Rac2.

The transduced HCS or hematopoietic progenitor cell is then transferred back to the patient.

The invention is further illustrated by the following examples and figures, from which further embodiments and advantages can be drawn. These examples are meant to illustrate the invention but not to limit its scope.

### Description of the figures

- Figs. 1 and 2: show certain embodiments of the vector for use in the invention.
- Fig. 3: shows that miR223 driven p47phox expression upon gammaretroviral gene therapy restores ROS production and *E. coli* killing in human granulocytes;
- Fig. 4: shows restoration of ROS production in human macrophages of a p47phox-deficient CGD patient after transduction of patient-derived monocytes upon lentiviral transduction of monocytes;
- Fig. 5: DNA methylation of the silencing sensitive SFFV enhancer/promoter (CpG island) as well as of a part of the p47phox transgene in human CD34+ cells after four weeks in cell culture. CD34+ cells were transduced gamma-retrovirally and cultured for four weeks. DNA was isolated treated with sodium bisulfite, amplified in a PCR reaction and sequences thereafter. Every horizontal line represents a sequencing result in which the information content was reduced to CpG dinucleotides: empty circles = unmethylated CpGs; black circles = methylated CpGs.
- Fig 6: miR223 driven GFP expression in CD11 b+ cells upon transduction of human HSCs of a healthy donor with a lentiviral vector encoding green fluorescent protein (GFP) under control of the miR223 promoter or the SFFV promoter, followed by myeloid differentiation to CD11 b+ cells in liquid cell culture.

### Examples

The present invention is particularly based on the surprising finding that use of the miR223 promoter in gene therapy vectors advantageously overcomes known problems of transactivation and/or epigenetic inactivation. This was particularly shown by LV-SIN gene therapy vector for autosomal-recessive p47phox-deficient CGD. Of note, lentiviral transduction of human HSCs with a lentiviral vector encoding p47phox under control of the miR223 promoter for p47phox CGD treatment was never shown before.

### Vectors utilized pre-clinically to characterize transgene expression

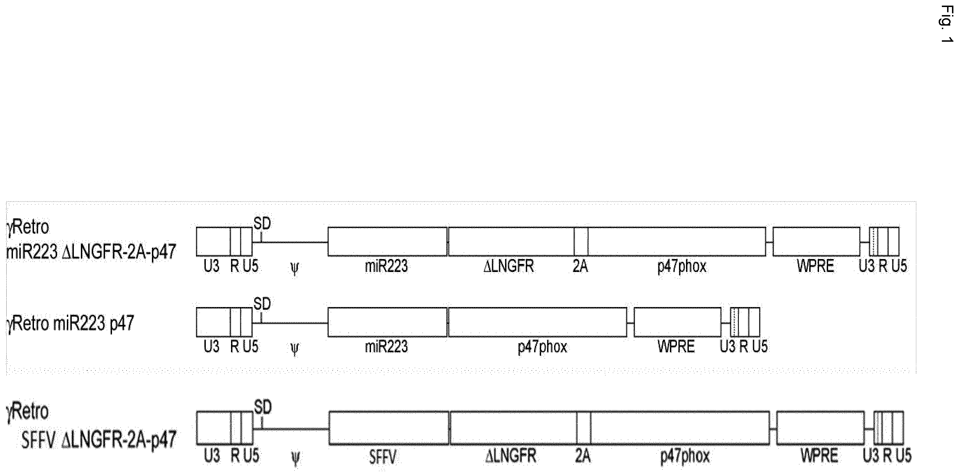

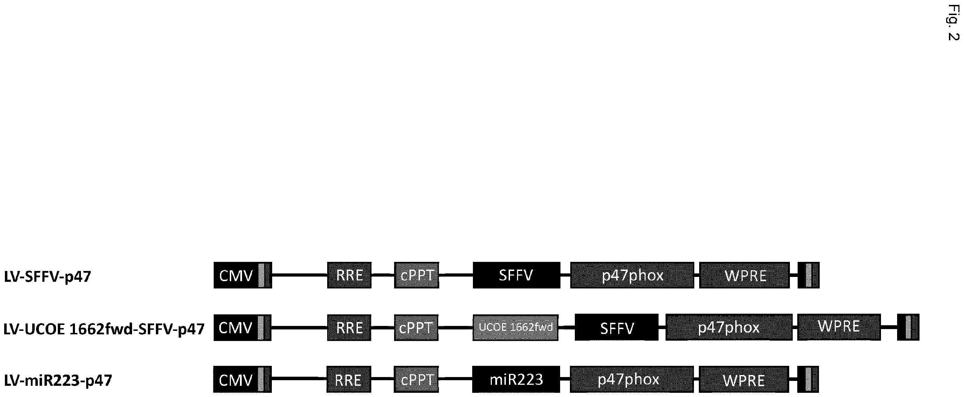

Data confirming a highly myelospecific transgene expression were generated with gamma-retroviral vectors and lentiviral vectors. Examples for utilized gamma-retroviral vectors are shown in Fig. 1. The ΔLNGFR-2A-p47phox fusion construct results in two separate proteins (cytoplasmic p47phox, and ΔLNGFR on the cell surface) both derived from one mRNA (v). Examples for utilized lentiviral vectors are shown in Fig. 2.

### Prevention of transactivation in HSCs by limiting expression to myeloid cells

### Myelospecificity of miR223 promoter activity shown in animal studies

Lineage-negative bone marrow cells were isolated from p47phox -/- mice and transduced with gamma-retroviral vectors encoding p47phox or the ΔLNGFR-2A-p47 fusion construct under miR223 promoter control (Figure 1). Recipient p47 -/- mice were lethally irradiated, and re-infused intravenously with transduced bone marrow cells. Six weeks after re-infusion, p47phox expression and ROS production were restored in granulocytes, indicating successful engraftment of transduced bone marrow cells. (Brendel et al. (2013) Hum Gene Ther Methods. 24: 151-159).

In parallel, lineage negative bone marrow cells were isolated from gp91phox-/- mice and transduced with a LV-SIN vector encoding gp91phox under miR223 promoter control. Recipient gp91phox -/- mice were lethally irradiated, and re-infused intravenously with transduced bone marrow cells. Transduced cells engrafted, leading to transgene expression and restoration of ROS production in peripheral granulocytes of these mice (Brendel et al. (2013) *ibid.).*

Analysis of transgene expression revealed an extraordinary lineage specificity conferred by the miR223 promoter in both animal experiments limiting transgene expression almost exclusively to myeloid cells (Brendel et al. (2013) *ibid.).*

### Restoration of phagocyte function by miR223-driven transgene expression in primary derived patient material

In above mentioned animal experiments, miR223 driven gp91phox expression (lentiviral vector) as well as miR223 driven p47phox expression (gamma-retroviral vector) both restored ROS production in phagocytes upon gene therapy treatment in the corresponding animal models.

Reconstitution of ROS production and reconstitution of *E.coli* killing was tested in human granulocytes after treatment of HSCs with a gamma-retroviral vector and differentiation of cells in cell culture. For detection of cytoplasmic p47phox transgene expression in living granulocytes, the inventors generated a gamma-retroviral vector encoding a fusion construct consisting of the ΔLNGFR surface marker, linked to p47phox via 2A sequence from food and mouth disease virus. This fusion construct leads to synthesis of two proteins from one mRNA transcript, in one translation process: the cytoplasmic p47phox protein and the ΔLNGFR surface marker. This co-expression allows for indirect detection of p47phox by ΔLNGFR surface staining (Wohlgensinger et al. (2010) Gene Ther. 17: 1193-1199). In the gamma-retroviral vector utilized, the inventors expressed this fusion construct either under control of the constitutively active SFFV promoter, or of the miR223 promoter.

The inventors transduced human HSC of a p47phox-deficient CGD patient with these gammretroviral vectors, followed by differentiation of the cells to granulocytes in cell culture. Differentiation was confirmed by FACS measurement of granulocytic CD11b surface marker expression. Detection of ΔLNGFR surface marker indirectly indicated p47phox expression.

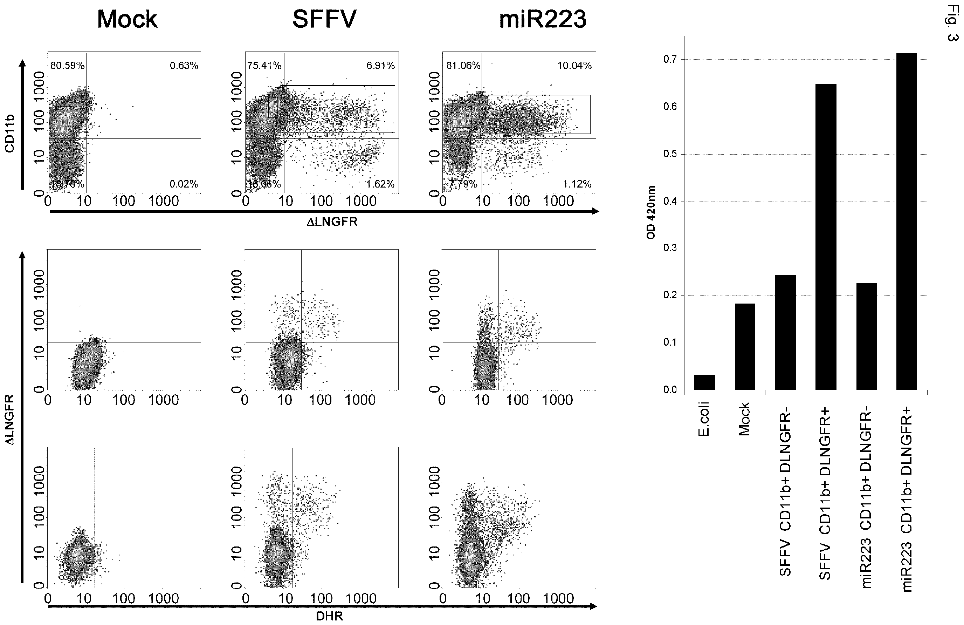

ROS production was detected by a dihydrorhodamine 123 oxidation (DHR) assay upon stimulation of cells with PMA or with *E. coli.* ROS production in *ex vivo* differentiated granulocytes was observed only in ΔLNGFR-positive cells (Figure 3).

Differentiated (CD11b+) and ΔLNGFR-positive granulocytes, and differentiated and ΔLNGFR-negative (non-transduced) cells were isolated by FACS-sorting, and applied in an *E. coli* killing assay as described (Ott et al. (2006) Nat Med 12: 401-409). In this assay, successful *E. coli* killing results in a rise in OD420nm. Importantly, using the gamma-retroviral SIN p47phox miR223 vector, p47phox transgene expression, ROS production and *E. coli* killing were restored to similar amounts as in a gamma-retroviral SIN p47phox vector in which transgene expression is driven by the strong constitutively active SFFV promoter (Figure 3).

Figure 3 shows that miR223-driven p47phox expression restores ROS production and *E. coli* killing in human granulocytes. Lineage-negative HSCs of a p47phox CGD patient were gamma-retrovirally transduced with the ΔLNGFR/p47phox co-expression construct under SFFV or miR223 promoter control, and differentiated to CD11b+ granulocytes. ΔLNGFR/p47phox-expressing granulocytes were FACS sorted, and applied in an *E. coli* killing assay. A rise in OD420nm indicates the restoration of *E. coli* killing.

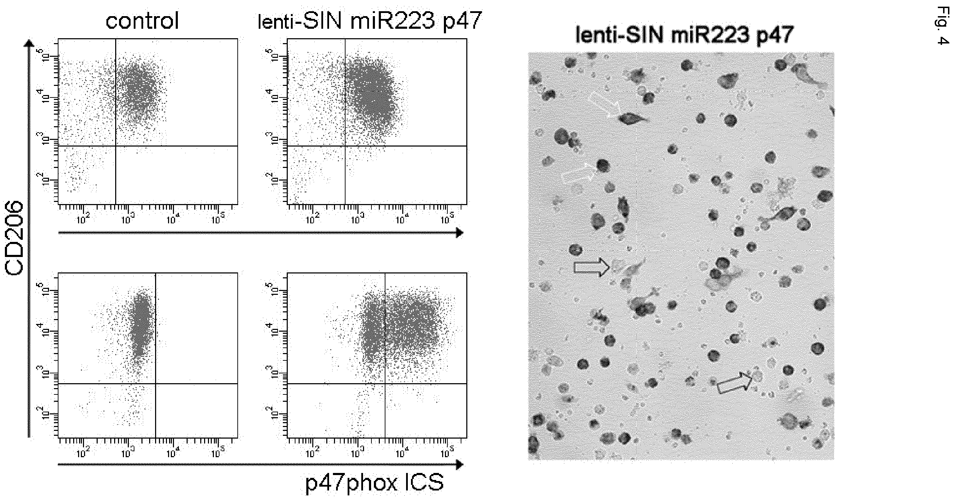

Restoration of phagocyte function was also shown in primary derived p47phox CGD patient material. The lenti_miR223_p47 WPREmut6 vector (Figs. 2 and 4) was used to transduce monocytes from a p47phox-deficient CGD patient. Transduced monocytes were differentiated to macrophages for seven days resulting in CD14+/CD206+ macrophages. p47phox transgene expression was confirmed by intracellular FACS staining, and ROS production upon PMA stimulation was confirmed in a nitroblue teterazolium (NBT) reduction assay (Fig. 4).

Figure 4 illustrates the restoration of ROS production in human macrophages of a p47phox-deficient CGD patient. Monocytes of a p47phox CGD patient were isolated from blood, transduced with the LV-SIN lenti_miR223_p47 WPREmut6 vector, and differentiated to macrophages. Differentiation to CD14+/CD206+ macrophages and restoration of p47phox expression were confirmed by FACS (left). Restoration of ROS production upon PMA stimulation was confirmed by NBT assay. Yellow arrow: NBT-positive, i.e. ROS producing, macrophage, empty arrow: NBT-negative macrophage.

### Prevention of epigenetic inactivation of transgene expression

### Kinetics of epigenetic inactivation in vivo and in cell culture

In the first temporarily successful clinical X-CGD gene therapy study, transgene expression was driven by the gammaretroviral promoter/enhancer within the viral LTR. Epigenetic inactivation of transgene expression started about one year after gene therapy, manifesting in an increasing discrepancy between gene marking and gene function within the 1.5 years thereafter (Siler et al. (2015) Current Gene Therapy 15: 416-427). The sequence of the promoter/enhancer within the LTR of the utilized gamma-retroviral vector corresponds to the SFFV promoter/enhancer utilized in various vectors as ubiquitously active promoter. This SFFV promoter was tested for its susceptibility to epigenetic modification in CGD animals three and six months after gene therapy treatment. Epigenetic SFFV promoter inactivation by DNA methylation in mice was already detectable after three months and increased thereafter over time (Zhang et al. (2010) Mol Ther 18: 1640-49).

Cells in a very primitive differentiation state like embryonic carcinoma cell lines (He et al. (2005) J Virol 79: 13497-508), embryonic stem cells (Liew et al. (2007) Stem Cells 25: 1521-28) and induced pluripotent stem cells (iPSCs) (Hotta & Ellis (2008) J Cell Biochem 105: 940-8) are known for their high DNA methylation activity. Epigenetic inactivation of the SFFV promoter within a lentiviral vector by P19 embryonic carcinoma cells is already detectable six to 12 days after transduction of the cells (Zhang et al. (2010) *ibid.).*

### Novel anti-silencing activity of the miR223 promoter

The inventors tested the susceptibility of the miR223 promoter to DNA methylation in P19 cells and in a p47phox iPSC cell line.

Within a lentiviral vector (Figure 2) an average 57.1% of CpGs within the miR223 promoter and more than 70% of CpGs within the SFFV promoter were methylated in P19 cells after 20 days (unpublished data). As P19 cells cannot be differentiated into the myeloid lineage (to phagocytes) and as the miR223 promoter is a myelospecific promoter, the inventors tested their lentiviral vector encoding p47phox under control of the miR223 promoter in p47phox CGD patient derived iPSCs (iPSC CGD1.1; Jiang et al. (2012) Stem Cells 30: 599-611) as these cells possess a high DNA methylation activity and can be propagated to phagocytes. iPSC CGD1.1 cells were transduced with a lentiviral vector encoding p47phox under control of the miR223 promoter (see Table 1 below) at multiplicity of infection 2 (MOI 2, i.e. twice as many viral particles as cells).

Starting day +13 post-transduction, iPSCs were propagated first to "embryoid bodies" and further to monocyte-releasing "monocyte factories". First monocytes could then be harvested starting day+43 post transduction. Harvested monocytes were further differentiated to macrophages by seven days' incubation in medium supplemented with M-CSF.

Average gene therapy vector copy number per genome (VCN) was determined by qPCR on day+16 in iPSCs as well as on day+85 in harvested monocytes. p47phox expression was quantified by flow cytometry (FACS) analysis on day+6, +10, +16, +20 in iPSCs, on day+23 in CD133+ stem cells within embryoid bodies, in CD38dim/CD34+ cells in embryoid bodies on day+104, in CD14+ cells on day+52, in CD15+ cells on day+48, in CD206+ cells on day+68. The percentage of p47phox-positive cells per vector copy number and the FACS derived mean fluorescent intensity (MFI) of p47phox-positive cells were determined to compare miR223 promoter activity in individual cell populations.

p47phox expression analysis in various differentiation stages revealed that in the average on stem cell level (iPSCs, CD133+ cells, CD34+ cells) (n=6), miR223-driven p47phox expression was barely detectable, with the percentage of p47-positive cells per vector copy number of 0.67+/-0.33(Stdev). Upon myeloid differentiation, p47phox expression strongly increased, with a percentage of p47-positive cells per vector copy number of 5.26+/-0.66. This rise in the percentage of p47phox expressing cells ran in parallel with a strong increase in FACS signal intensity (MFI), strongly indicating an activation of the miR223 promoter upon differentiation from stem cell level to myeloid cells.

Gene therapy mediated restoration of p47phox production in granulocytes, monocytes and macrophages generated from transduced p47phox CGD iPSCs resulted in the restoration of ROS production as revealed in a Dihydrorhodamine 123 oxidation (DHR) assay and in a nitroblue tetrazolium reduction (NBT) assay.

In parallel with the analysis of miR223 promoter activity on various differentiation levels between iPSCs and myeloid cells (granulocytes, monocytes, macrophages), DNA methylation analysis of the miR223 promoter within the gene therapy vector and of the endogenous miR223 promoter gene within the genome of transduced cells was performed in iPSCs on day+20 post transduction, and in monocytes obtained from transduced iPSCs upon differentiation on day+85 post iPSC transduction. Analysis of iPSCs and iPSC-derived monocytes upon transduction with vector comprising p47phox under control of the silencing-sensitive SFFV promoter served as control.

This analysis (Table 1) revealed that, in parallel with P19 cells on stem cell level, the vast majority (>80%) of CpG dinucleotides of the miR223 promoter were methylated, within the gene therapy vector encoded miR223 promoter, as well as within the native endogenous miR223 gene promoter in the genome of the transduced cells. On a functional level, the methylated miR223 promoter was inactive, as was shown by p47phox expression analysis.

**Table 1: DNA methylation analysis of the gene therapy vector encoded miR223 promoter and of the native miR223 gene promoter on iPSC (induced progenitor stem cell) level (day+20) and in iPSC-derived monocytes day+85 post iPSC transduction. A vector encoding the silencing-sensitive SFFV promoter served as control. Change in miR223 promoter DNA demethylation was statistically significant.**

| Promoter DNA methylation analysis in iPSCs (day+20) and iPSC-derived monocytes (day +85) after transduction of iPSC with a lentiviral vector | | | |
|---|---|---|---|
| % methylated CpGs +/-SEM | Viral encoded miR223 | Endogenous miR223 gene promoter | SFFV promoter |
| In iPSCs | 86.8% +/-4.8 | 80.0 +/-7.5 | 72.4 +/-9.7 |
| In monocytes | 31.3 +/-4.0 | 20.8 +/-7.4 | 70.6 +/-6.3 |
| p-value (iPSC vs monoctyes | <0.001 | <0.001 | >0.05 |

Surprisingly the inventors found that the initially methylated miR223 promoter within the analysed gene therapy vector and the native genomic miR223 gene promoter both became demethylated upon myeloid differentiation (Table 1).

In parallel to a rise in promoter activity, the percentage of CpG dinucleotide methylation within the miR223 promoter dropped from 87% to 31%.

In the inventors' previous gamma-retroviral X-CGD (gp91phox-deficient form of CGD) clinical gene therapy trial, transgene expression was driven by a constitutively active (SFFV) promoter. A gradual increase in gene therapy vector encoded promoter methylation caused clinically a gradual decrease in therapeutic efficacy over time (Ott et al. (2006) Nat Med 12: 401-409; Siler et al. (2015) Curr Gene Ther 15: 416-427). In this trial, the constitutively active promoter became methylated in HSC, and resulted in a release of HSC from bone marrow with methylated promoter within the gene therapy vector, and therefore with diminished therapeutic transgene expression.

Utilizing the myelospecific miR223 promoter as internal promoter within a gene therapy vector, the surprisingly observed methylation pattern indicates that the promoter is inactive and methylated at stem cell level. Only upon differentiation, the miR223 promoter is activated and demethylated. This strongly indicates that this promoter is able to provide long-term efficacy. As there is a continuous flux of myeloid cells derived from HSC that are released from bone marrow, in these newly produced cells the miR223 promoter actively became demethylated prior to release from bone marrow.

### Kinetics of promoter DNA methylation

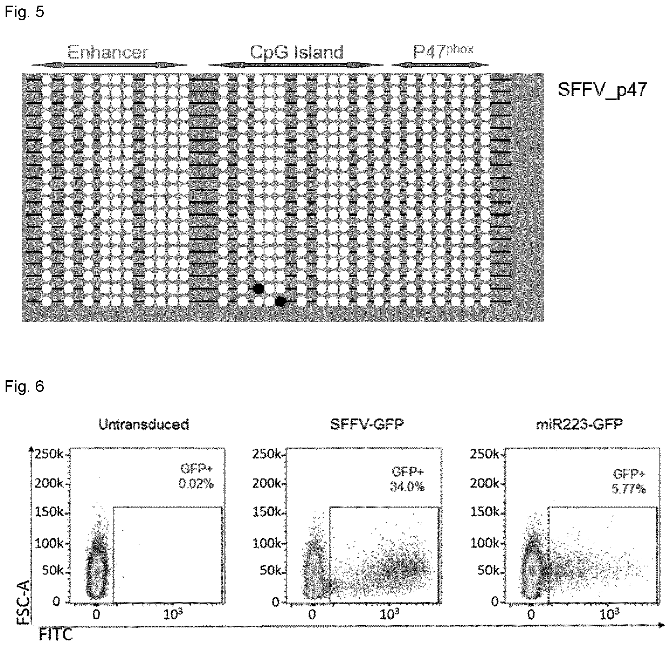

In a cell culture experiment, the inventors introduced the SFFV promoter in human CD34+ bone marrow cells by gamma-retroviral transduction, cultured the cells for four weeks and quantified DNA methylation thereafter. The inventors were unable to detect significant amounts of DNA methylation in human CD34+ cells after four weeks in cell culture (Figure 5).

Silencing of the SFFV promoter *in vivo* in mouse bone marrow was reported to become detectable three months after gene therapy, and to progress further in the following three months (Zhang et al. (2010) *ibid.).*

Retroviral vectors are reported to be immediately silenced in pluripotent stem cells including early preimplantation embryos, embryonic stem (ES) cells and embryonic carcinoma (EC) cells (Pannell D, Ellis J. (2001) Rev Med Virol 11: 205-217). The embryonic carcinoma cell line P19 has been intensively utilized to test the susceptibility of promoters within gene therapy vectors to silencing (Knight et al. (2012) J Virol 86: 9088-95; Brendel et al. (2012) Gene Therapy 19: 1018-29; Zhang, Santilli & Thrasher (2017) Sci Rep. 7: 10213; Zhang et al. (2010) *ibid.).* Silencing of the SFFV promoter is consistently reported in this model to be finished already after 12 to 17 days.

As P19 cells can't be differentiated, this model is not compatible to test tissue specific promoters. Meanwhile it is known that retroviral expression is silenced in induced pluripotent stem cells (iPSCs) (Maherali et al. (2007) Cell Stem Cell 1: 55-70; Okita, Ichisaka & Yamanaka (2007) Nature 448: 313-317; Wernig et al. (2007) Nature 448: 318-324), which have the capacity to be differentiated in cell culture into any cell type. iPSCs were generated from CGD patient material. It was shown that these iPSCs have the same genetic defect as was found in the patient material. The cells can be differentiated to mature monocytes and macrophages, and in contrast to monocytes and macrophages from non-CGD iPSCs, the CGD iPSC derived monocytes and macrophages show no production of reactive oxygen species (ROS), as is true for monocytes and macrophages from CGD patients (Jiang et al. (2012) Stem Cells 30:599-611).

Upon retroviral introduction of the SFFV promoter into CGD iPSCs, the SFFV promoter, which was silenced *in vivo* in human bone marrow in the clinical X-CGD trial starting after 10 months, in mouse bone marrow after 3 to 6 months, and which was silenced in P19 cells after 12 to 17 days, was silenced in CGD iPSCs within 20 days (Hänseler et al. (2018) Matters; doi.: 10.19185/matters.201805000005). Upon differentiation of SFFV promoter harboring CGD iPSCs, the SFFV promoter driven transgene expression was strongly diminished in terminally differentiated monocytes and macrophages. This shows that 1) iPSCs have a strong promoter silencing activity, 2) the SFFV promoter, once being silenced remains silenced upon cell differentiation from iPSCs to monocytes (Hänseler et al. (2018) Matters; doi.: 10.19185/matters.201805000005). iPSCs had been used for the development of systems, which prevent the silencing of ubiquitously active promoters like the SFFV promoter. These anti-silencing systems mainly based on the use of insulators (Sanchez-Hernandez et al. (2018) Mol Ther Nucleic Acids 13: 16-28) or on the use of the ubiquitous chromatin opening element (UCOE) (Pfaff et al. (2013) Stem Cells 31: 488-99; Ackermann et al. (2014) Biomaterials 35: 1531-42; Hoffmann et al. (2017) Gene Ther. 24: 298-307).

In summary, iPSCs are a relevant model for the analysis of gene therapy vector methylation, but this model had never been applied to analyze the susceptibility of tissue specific promoters to silencing.

### Analysis of the tissue specific miR223 promoter for its susceptibility to silencing

For the analysis of susceptibility to silencing of the miR223 promoter, the inventors utilized iPSCs, which were developed from a patient suffering from p47phox-deficient CGD (p47phox CGD iPSCs) and which were able to differentiate into monocytes and macrophages showing the CGD phenotype (Jiang et al. (2012) Stem Cells 30:599-611).

p47phox CGD iPSCs were lentivirally transduced (day+1) with the inventors' gene therapy vector (UZH1p47CGD), or with a lentiviral vector encoding p47phox under control of constitutively active and silencing resistant version of the SFFV promoter (Lenti UCOE 1662finid SFFV p47). Transduced iPSC were differentiated first to embryoid bodies (EB), then further to monocytes and macrophages. Average vector copy number per cell (VCN) was determined by qPCR in iPSC on day+16, and in monocytes on day+85 after transduction. Transgene expression was monitored in CD133-positive stem cells in embryoid bodies on day +23, in CD38dim/CD34+ cells within embryoid bodies (which are cells comparable to HSC) on day+104, in monocytes on day+52, and in macrophages on day+68 after transduction. p47phox expression was detected by FACS analysis upon intracellular staining of p47phox protein in combination with CD34, CD38, CD14 and CD206 surface staining. To correct for the differences in transduction efficiencies, percentages of p47phox-positive cells were divided by the VCN.

### p47phox transgene expression:

In iPSC and in the iPSC derived homolog of HSC, i.e. the CD38^{dim}/CD34^{high} cells within embryoid bodies (from now on in this context termed "HSCs"), the miR223 promoter within the inventors gene therapy vector UZH1p47CGD resulted in markedly weaker p47phox transgene expression, as measured by signal intensity, and a low percentage of p47phox-positive cells per vector copy number. In contrast, in differentiated monocytes and macrophages, signal intensity and percentage of p47phox-positive cells per vector copy number was high in transduced cells (see following Table 2).

**Table 2: shows the p47phox transgene expression in iPSCs and iPSC-derived cell populations characterized by percentage of p47phox-positive cells per vector copy number (CVN) and by flow cytometry-derived mean fluorescent signal intensity (MFI). Human p47phox-negative iPSCs were lenti-virally transduced and propagated via embryoid bodies to monocytes and to macrophages. Average vector copy number (VCN) was determined in iPSC, and in iPSC derived monocytes. Transgene expression was monitored in CD133+ stem cells, in HSC-like CD34+ cells within embryoid bodies, in monocytes, and in macrophages. p47phox expression was detected by FACS analysis upon intracellular staining. Percentages of p47phox-positive cells were normalized for transduction efficiencies by calculation of the percentage of p47-positive cells per VCN. miR223-driven p47phox expression showed a marginal expression in CD133+ and in CD34+ cells and a rise in expression upon differentiation as revealed by percent of p47phox-positive cells as well as in MFl.**

| Induction of p47phox expression upon differentiation | | in iPSC-derived CD133+ stem cells | in iPSC-derived HSC-like CD34+ cells | in iPSC-derived CD14+ monocytes | in iPSC-derived CD206+ macrophages |
|---|---|---|---|---|---|
| % p47+/VCN | SFFV | 14.7 | 3.4 | 6 | 4.6 |
| | miR223 | 0.6 | 0.2 | 5.5 | 5.7 |
| MFI of p47+ cells | SFFV | 0.96 | 3.56 | 1.56 | 1.8 |
| | miR223 | 0.57 | 1.93 | 2.39 | 3.75 |
| | CGD control | 0.48 | 3.09 | 1.12 | 1.22 |
| fold induction of MFI rel. to contr. | SFFV | 2 | 1.15 | 1.39 | 1.48 |
| | miR223 | 1.19 | 0.62 | 2.13 | 3.07 |

Importantly, the miR223 promoter driven p47phox expression led to a low background signal (percent positive cells/VCN) as well as expression strength (MFI) in stem cells. The low background signal in stem cells indicates an excellent safety profile of the UZH1p47CGD gene therapy vector.

Human HSCs of a healthy donor were transduced with a lentiviral vector encoding GFP under control of the SFFV or the miR223 promoter, followed by differentiation to CD11b-positive myeloid cells in liquid cell culture. Expression of GFP was analyzed in human HSC-derived CD11b-positive cells by FACS analysis. Detection of GFP-positive human HSC-derived myeloid cells (Fig. 6, unpublished data) was in line with vector copy number (CVN) of 0.05 and shows transgene expression activity of the miR223 promoter in human HSC-derived myeloid cells as required for CGD gene therapy treatment.

### Reconstitution of ROS production:

ROS production was monitored in monocytes and macrophages by DHR oxidation and by NBT reduction assay upon stimulation with PMA (monocytes) or with PMA+fMLP (macrophages). In the DHR assay, the non-fluorescent dihydrorhodamin turns fluorescent upon oxidation by ROS. Fluorescence signal intensities were detected by FACS analysis. Correspondingly, in FACS analysis a shift to the right indicates restoration of ROS production. In the NBT assay, the formation of dark blue formazan precipitates indicates ROS production. Monocytes/macrophages from untransduced CGD iPSCs served as negative controls. As positive control served monocytes/macrophages obtained from CGD iPSCs, which were transduced with a vector encoding p47phox under control of a ubiquitously active and silencing resistant version of the SFFV promoter (Lenti UCOE1662finid SFFV p47).
miR223 driven p47phox expression by the inventors' gene therapy vector UZH1p47CGD resulted in reconstitution of ROS production and therefore in the correction of the CGD phenotype, as visualized in both the DHR assay and in the NBT assay.

### Silencing (DNA methylation) analysis in the iPSC model:

The inventors utilized p47-/- iPSCs to test for methylation of their gene therapy vector (UZH1p47CGD) and for transgene expression activity upon iPSC differentiation to phagocytes. p47phox-negative iPSC were lenti-virally transduced and propagated to monocytes. DNA was isolated from transduced iPSC (day+20) and from iPSC-derived monocytes (d+97). For DNA methylation analysis, isolated DNA was exposed to sodium bisulfite, which converts only unmethylated cytosine to uracil. Subsequent PCR amplification converts uracil to thymidine. Comparison of the original sequence with the sequence after bisulfite conversion reveals DNA methylation, i.e. in all methylated sequences a cytosine is detected by sequencing, whereas in unmethylated sequences thymidine is detected.

DNA methylation analysis was conducted in iPSCs and iPSC-derived monocytes upon transduction with the gene therapy vector (UZH1p47CGD) encoding p47phox under miR223 control. Methylation analysis covered the whole miR223 promoter sequence, as well as a part of the p47phox transgene. In addition, the inventors analyzed DNA methylation of the native miR223 gene promoter sequence of the iPSC genome.

Importantly, in iPSC both the virally encoded miR223 promoter, as well as the native miR223 promoter, were almost completely methylated within 20 days. Accordingly, in CD133+ iPSCs and in iPSC-derived CD34+CD38- stem cells, p47phox transgene expression was extremely low (see Table 1 above).

Neither on iPSC level nor in monocytes, the degree of methylation between gene therapy vector encoded miR223 promoter sequence and native miR223 promoters, did not differ significantly. Surprisingly and in contrast to results obtained with the SFFV promoter (Hänseler et al. (2018) Matters; doi.: 10.19185/matters.201805000005), the inventors found that upon differentiation from iPSCs to terminally differentiated cells, methylation of both the viral and the native miR223 promoter dropped significantly, indicating active demethylation upon differentiation (Table 1) and thus activation (Table 2). Extrapolating to the long term *in vivo* situation, this finding indicates long term activity of the miR223 promoter, as being silenced in dormant HSC, the miR223 promoter is expected to become active upon HSC activation and differentiation. As mentioned earlier, UCOE based systems were developed, which can prevent the silencing of the SFFV, or of the Chim promoter (Zhang, Santilli & Thrasher (2017) Sci Rep 7: 10213). But the UCOE system embedded into a retroviral gene therapy vector harbors the risk of altering the DNA methylation of DNA in proximity to the retroviral integration site, also in HSC. This activity might in HSC activate oncogenes close to the integration site of the gene therapy vector in all consequence. In contrast to the UCOE system, the miR223 promoter within the vector UZH1p47CGD adapted to the surrounding methylation pattern in stem cells and only upon differentiation became demethylated, which is a significant safety feature.

## Claims

1. An isolated human hematopoietic stem cell or myeloid progenitor cell, transduced with a lentiviral vector, said lentiviral vector comprising
- a coding nucleic acid sequence encoding a functional variant of a polypeptide selected from gp91phox and p47phox; under transcriptional control of
- the miR223 promoter sequence **characterized by** SEQ ID NO 01.

2. The isolated human hematopoietic stem cell or myeloid progenitor cell according to claim 1, wherein said lentiviral vector comprises or essentially consists of a nucleic acid sequence **characterized by** SEQ ID NO 02.

3. The human hematopoietic stem cell or myeloid progenitor cell according to any one of claims 1 or 2, wherein the cell is a CD34 positive hematopoietic stem cell.

4. A method for preparing a therapeutic cell preparation, comprising the steps of:
a. providing a preparation of cells comprising hematopoietic stem cells, particularly a preparation of CD34 positive hematopoietic stem cells, isolated from a patient suffering from chronic granulomatous disease associated with a gene defect associated with a defective gene encoding a polypeptide selected from gp91phox and p47phox;
b. transducing said preparation of cells with a lentiviral vector comprising
i. a coding nucleic acid sequence encoding a functional variant of a polypeptide selected from gp91phox, and p47phox; under transcriptional control of
ii. the miR223 promoter sequence **characterized by** SEQ ID NO 01.

5. The isolated human hematopoietic stem cell or myeloid progenitor cell according to any one of claims 1 to 3, or a cell preparation obtained by a method according to claim 4, for use in treatment or prevention of chronic granulomatous disease.

6. The isolated human hematopoietic stem cell or myeloid progenitor cell for use in treatment or prevention of chronic granulomatous disease according to claim 5, wherein the cell is an autologous cell.

## Patentansprüche

1. Isolierte humane hämatopoetische Stammzelle oder isolierte humane myeloische Vorläuferzelle, die mit einem lentiviralen Vektor transduziert ist, wobei der lentivirale Vektor umfasst:
- eine codierende Nucleinsäuresequenz, die eine funktionelle Variante eines aus gp91phox und p47phox ausgewählten Polypeptids codiert; unter der transkriptionellen Kontrolle der
- durch SEQ ID NO:1 charakterisierten miR223-Promotorsequenz.

2. Isolierte humane hämatopoetische Stammzelle oder isolierte humane myeloische Vorläuferzelle nach Anspruch 1, wobei der lentivirale Vektor eine durch SEQ ID NO:2 charakterisierte Nucleinsäuresequenz umfasst oder im Wesentlichen aus dieser besteht.

3. Humane hämatopoetische Stammzelle oder humane myeloische Vorläuferzelle nach einem der Ansprüche 1 oder 2, wobei die Zelle eine CD34-positive hämatopoetische Stammzelle ist.

4. Verfahren zum Herstellen eines therapeutischen Zellpräparats, umfassend die Schritte:
a. Bereitstellen eines Zellpräparats, das hämatopoetische Stammzellen umfasst, insbesondere ein Präparat von CD34-positiven hämatopoetischen Stammzellen, die von einem Patienten isoliert sind, der an einer chronischen granulomatösen Erkrankung leidet, die mit einem Gendefekt in Zusammenhang steht, der mit einem defekten Gen, das ein aus gp91phox und p47phox ausgewähltes Polypeptid codiert, in Zusammenhang steht;
b. Transduzieren des Zellpräparats mit einem lentiviralen Vektor umfassend:
i. eine codierende Nucleinsäuresequenz, die eine funktionelle Variante eines aus gp91phox und p47phox ausgewählten Polypeptids codiert; unter der transkriptionellen Kontrolle der
ii. durch SEQ ID NO:1 charakterisierten miR223-Promotorsequenz.

5. Isolierte humane hämatopoetische Stammzelle oder isolierte humane myeloische Vorläuferzelle nach einem der Ansprüche 1 bis 3, oder ein Zellpräparat, das mit einem Verfahren nach Anspruch 4 erhalten wurde, zur Verwendung bei der Behandlung oder Vorbeugung einer chronischen granulomatösen Erkrankung.

6. Isolierte humane hämatopoetische Stammzelle oder isolierte humane myeloische Vorläuferzelle zur Verwendung bei der Behandlung oder Vorbeugung einer chronischen granulomatösen Erkrankung nach Anspruch 5, wobei die Zelle eine autologe Zelle ist.

## Revendications

1. Cellule souche hématopoïétique ou cellule progénitrice myéloïde humaine isolée, transduite avec un vecteur lentiviral, ledit vecteur lentiviral comprenant
- une séquence d'acides nucléiques codante codant pour un variant fonctionnel d'un polypeptide choisi parmi gp91phox et p47phox ; sous contrôle transcriptionnel de
- la séquence de promoteur miR223 **caractérisée par** la SEQ ID NO: 1.

2. Cellule souche hématopoïétique ou cellule progénitrice myéloïde humaine isolée selon la revendication 1, ledit vecteur lentiviral comprenant ou étant essentiellement constitué d'une séquence d'acides nucléiques **caractérisée par** la SEQ ID NO: 2.

3. Cellule souche hématopoïétique ou cellule progénitrice myéloïde humaine isolée selon l'une quelconque des revendications 1 ou 2, la cellule étant une cellule souche hématopoïétique positive pour CD34.

4. Procédé pour la préparation d'une préparation cellulaire thérapeutique, comprenant les étapes de :
a. fourniture d'une préparation de cellules comprenant des cellules souches hématopoïétiques, particulièrement une préparation de cellules souches hématopoïétiques positives pour CD34, isolées d'un patient souffrant d'une maladie granulomateuse chronique associée à un défaut génétique associé à un gène défectueux codant pour un polypeptide choisi parmi gp91phox et p47phox ;
b. transduction de ladite préparation de cellules avec un vecteur lentiviral comprenant
i. une séquence d'acides nucléiques codante codant pour un variant fonctionnel d'un polypeptide choisi parmi gp91phox et p47phox ; sous contrôle transcriptionnel de
ii. la séquence de promoteur miR223 **caractérisée par** la SEQ ID NO: 1.

5. Cellule souche hématopoïétique ou cellule progénitrice myéloïde humaine isolée selon l'une quelconque des revendications 1 à 3, ou préparation cellulaire obtenue par un procédé selon la revendication 4, pour une utilisation dans le traitement ou la prévention d'une maladie granulomateuse chronique.

6. Cellule souche hématopoïétique ou cellule progénitrice myéloïde humaine isolée pour une utilisation dans le traitement ou la prévention d'une maladie granulomateuse chronique selon la revendication 5, la cellule étant une cellule autologue.
